# EUROPEAN PATENT SPECIFICATION

(11) **EP 2 344 156 B1**
(45) Date of publication and mention of the grant of the patent: **17.10.2018**
(21) Application number: 09780134.4
(22) Date of filing: 03.07.2009
(51) Int. Cl.: A61K 31/496, A61K 31/513, A61K 31/7068, A61K 31/4745, A61K 31/337, A61K 31/282, A61P 35/00

(54) **THERAPEUTIC COMBINATION COMPRISING AN AURORA KINASE INHIBITOR AND AN ANTINEOPLASTIC AGENT**
THERAPEUTISCHE KOMBINATION MIT EINEM AURORA-KINASE-INHIBITOR UND EINEM ANTINEOPLASTISCHEN MITTEL
COMBINAISON THÉRAPEUTIQUE COMPOSÉE D UN INHIBITEUR DE KINASE AURORA ET D UN AGENT ANTINÉOPLASIQUE

(30) Priority: 24.07.2008 US 83232 P
(43) Date of publication of application: 20.07.2011
(73) Proprietor: Nerviano Medical Sciences S.r.l., 20014 Nerviano (MI) (IT)
(72) Inventor: PESENTI, Enrico, 20015 Parabiago (MI) (IT); D'INCALCI, Maurizio, 20123 Milan (IT); BALLINARI, Dario, 20097 San Donato Milanese (MI) (IT); MOLL, Juergen, 22070 Appiano Gentile (CO) (IT)
(86) International application number: PCT/EP2009/058413
(87) International publication number: WO 2010/009967

(56) References cited:
- WO-A-2005/005427
- WO-A-2007/129062
- WO-A-2008/052931
- WO-A-2008/135232
- WO-A-2009/026075
- WO-A-2009/091476

## Description

### FIELD OF THE INVENTION

The present invention relates in general to the field of cancer treatment and, more particularly, provides an anti-tumor composition comprising an aurora kinase inhibitor and a platinum derivative and/or an antimetabolite agent and/or a topoisomerase I inhibitor and/or an antimicrotubule agent having a synergistic or additive antineoplastic effect.

### BACKGROUND OF THE INVENTION

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis, glomerulonephritis and post-surgical stenosis and restenosis.

PKs are also implicated in inflammatory conditions and in the multiplication of viruses and parasites. PKs may also play a major role in the pathogenesis and development of neurodegenerative disorders.

For a general reference to PKs malfunctioning or disregulation see, for instance, Current Opinion in Chemical Biology, 1999, 3, 459-465.

Among the several protein kinases known in the art as being implicated in the growth of cancer cells are Aurora kinases, in particular Aurora-2.

Aurora-2 was found to be over-expressed in a number of different tumour types. Its gene locus maps at 20q13, a chromosomal region frequently amplified in many cancers, including breast [Cancer Res. 1999, 59(9), 2041-4] and colon cancers.

20q13 amplification correlates with poor prognosis in patients with node-negative breast cancer and increased Aurora-2 expression is indicative of poor prognosis and decreased survival time in bladder cancer patients [J. Natl. Cancer Inst., 2002, 94(17), 1320-9]. For a general reference to the Aurora-2 role in the abnormal centrosome function in cancer see also Molecular Cancer Therapeutics, 2003, 2, 589-595.

Several heterocyclic compounds are known in the art as protein kinase inhibitors. Among them, 3-carboxamido-pyrazoles and 3-ureido-pyrazoles and derivatives thereof, have been disclosed as protein kinase inhibitors in the international patent applications WO01/12189, WO12188, WO02/48114 and WO02/70515.

Fused bicyclic compounds comprising pyrazole moieties and possessing kinase inhibitory activity have been also disclosed in WO00/69846, WO02/12242, WO03/028720 and WO03/97610.

Specific tetrahydropyrrolo[3,4-c]pyrazole derivatives have been revealed to be potent ATP-competitive inhibitors of Aurora kinases (Fancelli, D., et al.: Journal of Medicinal Chemistry. 2005, vol.48, no.8, p.3080-3084. PCT/WO 2005005427).

WO2007/129062 discloses a combination comprising a pyrazole compound that inhibits or modulates the activity of cyclin dependent kinases (CDK) and/or glycogen synthase kinase (e.g. GSK-3) and an anti-cancer agent (including a tetrahydropyrrolo[3,4-c]pyrazole derivative of the present invention).

WO2009/026075 describes a combination of antagonists of the hedgehog signalling pathway with a BCR-ABL inhibitor (including a tetrahydropyrrolo[3,4-c]pyrazole derivative of the present invention), possibly further comprising one or more chemotherapeutic or immunotherapeutic agents.

WO2009/091476 provides a combination comprising an ATP-competitive BCR-ABL inhibitor (including a tetrahydropyrrolo[3,4-c]pyrazole derivative of the present invention) and a non-ATP competitive BCR-ABL inhibitor, possibly further comprising one or more chemotherapeutic or immunotherapeutic agents.

Surprisingly, it has been found that the antineoplastic effect, i.e. especially the delay of progression or treatment of a proliferative disease, in particular the treatment of a tumour that is refractory to other chemotherapeutics known as antineoplastic agents, of the tetrahydropyrrolo[3,4-c]pyrazole derivatives above is greatly enhanced when they are administered in combination with certain antineoplastic agents. In particular, the antineoplastic effect of such combination is greater than the effects that can be achieved with either type of combination component alone, i.e. greater than the effects of a monotherapy using only one of the combination components.

The present invention thus provides new combinations of a tetrahydropyrrolo[3,4-*c*]pyrazole derivative with known pharmaceutical agents that are particularly suitable for the treatment of proliferative disorders, especially cancer. More specifically, the combinations of the present invention are very useful in therapy as antitumor agents and lack, in terms of both toxicity and side effects, the drawbacks associated with currently available antitumor drugs.

### SUMMARY OF THE INVENTION

The present invention provides, in a first aspect, a therapeutic combination consisting of (a) Compound 1 of formula (A): 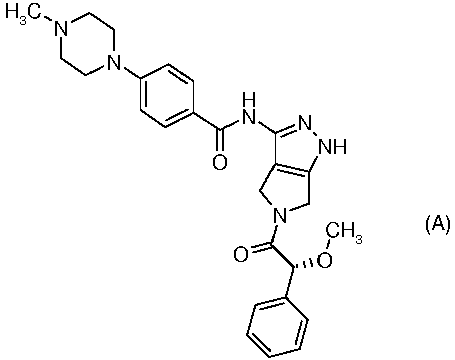 and (b) one or more antineoplastic agents selected from the group consisting of cisplatin, oxaliplatin, 5-fluorouracil, gemcitabine, cytosine arabinoside (Ara-C), SN-38, irinotecan and docetaxel, wherein the active ingredients of the combination are present in each case in free form or in the form of a pharmaceutically acceptable salt or any hydrate thereof.

The present invention also provides a therapeutic combination as described above for use in a method of treating or delaying the progression of a proliferative disorder, wherein the said method comprises a simultaneous, sequential or separate administration to a patient in need thereof of a therapeutically effective amount of the said combination.

The present invention also provides a pharmaceutical composition comprising a combination as described above which is further admixed with a pharmaceutically acceptable carrier, diluent or excipient.

### DETAILED DESCRIPTION OF THE INVENTION

The present invention provides, in a first embodiment, a therapeutic combination consisting of (a) Compound 1 of formula (A): 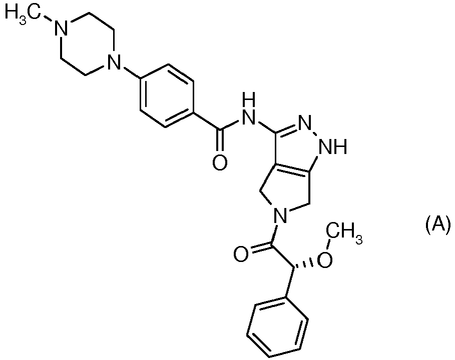 and (b) one or more antineoplastic agents selected from the group consisting of cisplatin, oxaliplatin, 5-fluorouracil, gemcitabine, cytosine arabinoside (Ara-C), SN-38, irinotecan and docetaxel, wherein the active ingredients of the combination are present in each case in free form or in the form of a pharmaceutically acceptable salt or any hydrate thereof.

A further embodiment relates to the combination according to the invention for use in a method of treating or delaying the progression of a proliferative disorder, wherein the said method comprises a simultaneous, sequential or separate administration to a patient in need thereof of a therapeutically effective amount of the said combination.

In a still further embodiment the invention provides a pharmaceutical composition comprising a therapeutic combination admixed with a pharmaceutically acceptable carrier, diluent or excipient.

The compound 1 of formula (A) has the chemical name N-{5-[(2R)-2-methoxy-2-phenylethanoyl]-1,4,5,6-tetrahydropyrrolo[3,4-c]pyrazol-3-yl}-4-(4-methylpiperazin-1-yl)benzamide. It can be prepared as described in WO2005/005427 (incorporated herein by reference), is endowed with protein kinase inhibitory activity and is thus useful in therapy as antitumor agent.

Pharmaceutically acceptable salts of compound 1 of formula (A) include the acid addition salts with inorganic or organic acids, e.g., nitric, hydrochloric, hydrobromic, sulphuric, perchloric, phosphoric, acetic, trifluoroacetic, propionic, glycolic, lactic, oxalic, malonic, malic, maleic, mesylate, tartaric, citric, benzoic, cinnamic, mandelic, methanesulphonic, isethionic and salicylic acid and the like.

Oxaliplatin can be administered, e.g., in the form as it is marketed, e.g. under the trademark ELOXATIN®.

Gemcitabine can be administered, e.g., in the form as it is marketed, e.g. under the trademark GEMZAR®.

Irinotecan can be administered, e.g., in the form as it is marketed, e.g. under the trademark CAMPTOSAR®. SN-38 is the active metabolite of irinotecan, obtained by *in vivo* hydrolysis of irinotecan.

Docetaxel can be administered, e.g., in the form as it is marketed, e.g. under the trademark TAXOTERE®.

In the present invention, each of the active ingredients of the combination is provided in an amount effective to produce a synergistic antineoplastic effect.

By the term "a synergistic antineoplastic effect" as used herein is meant the inhibition of the growth of the tumor, preferably the complete regression of the tumor, by administering an effective amount of the combination of compound 1 of formula (A) as defined above and cisplatin, oxaliplatin, 5-fluorouracil, gemcitabine, cytosine arabinoside (Ara-C), SN-38, irinotecan or docetaxel to mammals, including human.

The combination components (a) and (b) as defined above can be dosed independently or by use of different fixed combinations with distinguished amounts of the combination components (a) and (b), i.e. simultaneously or at different time points. The components can then, e.g., be administered simultaneously or chronologically staggered, that is at different time points and with equal or different time intervals for any component. Most preferably, the time intervals are chosen such that the effect on the treated disease in the combined use of the parts is greater than the effect which would be obtained by use of only any one of the combination components (a) and (b). The ratio of the total amounts of the combination component (a) to the combination component (b) to be administered in the combined preparation can be varied, e.g. in order to cope with the needs of a patient subpopulation to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, less toxicity, or a combined therapeutic effect in a non-effective dosage of one or both of the combination components (a) and (b), and very preferably a strong synergism of the combination components (a) and (b).

By the term "administered" or "administering" as used herein is meant parenteral and /or oral administration. By "parenteral" is meant intravenous, subcutaneous and intramuscular administration.

In the method of the subject invention, for the administration of the compound of formula (A), the course of therapy generally employed is in the range from 100 mg/m²/day to 1500 mg/m²/day of body surface area for up to 21 consecutive days. More preferably, the course therapy employed is from about 150 mg/m²/day to about 350 mg/m²/day of body surface area for up to 21 consecutive days.

In a particularly preferred regimen, the compound of formula (A) is administered in a dose of 190 or 250 mg/m²/day of body surface area for three hours infusion on day 1 and 8 of a three weeks cycle. Other possible therapeutic schedules are disclosed, for example, in WO2008/052931 published May 8, 2008 (incorporated herein by reference).

The compound of formula (A) can be administered in a variety of dosage forms, e.g., orally, in the form of tablets, capsules, sugar or film coated tablets, liquid solutions or suspensions; rectally in the form of suppositories; parenterally, e.g., intramuscularly, or through intravenous and/or intrathecal and/or intraspinal injection or infusion.

In the method of the subject invention, for the administration of oxaliplatin, the course of therapy generally employed is from 10 mg/m²/day to 100 mg/m²/day every 2-3 weeks. More preferably, the course of therapy generally employed is from about 30 mg/m²/day to 85 mg/m²/day on day 1, once every 2 weeks.

For the administration of gemcitabine, the course of therapy generally employed is from 200 mg/m²/day to 2000 mg/m²/day as weekly administration. More preferably, the course of therapy generally employed is from about 800 mg/m²/day to 1250 mg/m²/day on days 1 and 8 of a 21-day cycle or on days 1, 8 and 15 of a 28-day cycle.

For the administration of irinotecan, the course of therapy generally employed is from 50 mg/m² to 350 mg/m² daily on days 1, 8, 15 and 22 of a 6-week cycle. More preferably, the course of therapy generally employed is from about 125 mg/m² to 180 mg/m² daily on days 1, 8, 15 and 22 of a 6-week cycle.

For the administration of docetaxel, the course of therapy generally employed is from about 50 mg/m²/day to 100 mg/m²/day every three weeks or from 20 mg/m²/day to 100 mg/m²/day weekly. Preferably, administration occurs over 3 hours infusion on days 1 and 8 of a three- week cycle.

The antineoplastic therapy of the present invention is in particular suitable for treating all forms of cancer including, but not limited to: carcinoma such as bladder, breast, colon, kidney, liver, lung, including small cell lung cancer, esophagus, gall-bladder, ovary, pancreas, stomach, cervix, thyroid, prostate, and skin, including squamous cell carcinoma; haematopoietic tumors of lymphoid lineage including leukaemia, acute lymphocytic leukaemia, acute lymphoblastic leukaemia, B-cell lymphoma, T-cell-lymphoma, Hodgkin's lymphoma, non-Hodgkin's lymphoma, mantle cell lymphoma, hairy cell lymphoma and Burkett's lymphoma; haematopoietic tumors of myeloid lineage, including acute and chronic myelogenous leukemias, myelodysplastic syndrome and promyelocytic leukaemia; multiple myeloma; tumors of mesenchymal origin, including fibrosarcoma and rhabdomyosarcoma; tumors of the central and peripheral nervous system, including astrocytoma neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.

As stated above, the effect of the combination of the invention is significantly increased without a parallel increased toxicity. In other words, the combined therapy of the present invention enhances the antitumoral effects of the component (a) and/or of component (b) of the combination of the invention and thus yields the most effective and less toxic treatment for tumors.

Pharmaceutical compositions according to the invention are useful in anticancer therapy.

The present invention further discloses a commercial package comprising, in a suitable container means, (a) a compound of formula (A) as defined above, and (b) one or more antineoplastic agent selected from the group consisting of cisplatin, oxaliplatin, 5-fluorouracil, gemcitabine, cytosine arabinoside (Ara-C), SN-38, irinotecan or docetaxel wherein the active ingredients are present in each case in free form or in the form of a pharmaceutically acceptable salt or any hydrate thereof, together with instructions for simultaneous, separate or sequential use thereof.

In a package according to the invention each of partner (a) and (b) are present within a single container means or within distinct container means.

Due to the key role of the aurora kinase proteins in the regulation of cellular mitosis and proliferation, the combinations of the present invention are also useful in the treatment of a variety of cell proliferative disorders such as, for example, benign prostate hyperplasia, familial adenomatosis, polyposis, neurofibromatosis, psoriasis, vascular smooth cell proliferation associated with atherosclerosis, pulmonary fibrosis, arthritis, glomerulonephritis and post-surgical stenosis and restenosis.

The combinations of this invention, as modulators of apoptosis, may also be useful in the treatment of cancer, viral infections, prevention of AIDS development in HIV-infected individuals, autoimmune diseases and neurodegenerative disorders.

The activities of the combination of the present invention are shown for instance by the following *in vitro* and *in vivo* tests, which are intended to illustrate but not to limit the present invention.

### Example 1: Materials and methods for in vitro cytotoxic activity testing

Exponentially growing human ovarian carcinoma (A2780) and/or human colon carcinoma (HCT-116) cell lines were seeded and incubated at 37°C in a humidified 5% CO₂ atmosphere. Drugs were added to the experimental culture, and incubations were carried out at 37° C for 72 hours in the dark. Scalar doses of Compound 1 of formula (A) as defined above and antineoplastic agent were added to the medium 24 hours after seeding. One or both of the following treatment schedules were tested: simultaneous administration (both drugs administered to cells for 72 hours) and sequential administration (Compound 1 administered 24 hours after the other agent). Drug solutions were prepared immediately before use. At the end of treatment, cell proliferation was determined by an intracellular adenosine triphosphate monitoring system (CellTiterGlo-Promega) using the Envision (PerkinElmer) as reader. Inhibitory activity was evaluated comparing treated versus control data using Assay Explorer (MDL) program. The dose inhibiting 50% of cell growth was calculated using sigmoidal interpolation curve. Combination indices (C.I.) were calculated using a computer program for multiple drug effect analysis based on the equation of Chou-Talalay (Adv Enzyme Regul 1984;22:27-55) for mutually nonexclusive drugs, where a C.I. <1 indicates a more than additive effect (C.I. >3 indicates strong antagonism; 1.3<C.I.<3, antagonism; 0.8<C.I.<1.3, additivity; 0.3<C.I. < 0.8, synergism; C.I.<0.3, strong synergism).

### Example 2: In vitro cytotoxic activity of the combination with cisplatin

The results shown in Table 1 indicate that on both human ovarian carcinoma A2780 and human colon carcinoma HCT-116 cell lines the administration of Compound 1 in combination with cisplatin resulted in a synergistic antitumor effect.

| **Table 1.** *In vitro* evaluation of the cytotoxic effect of Compound 1 in combination with cisplatin | | | | | |
|---|---|---|---|---|---|
| Cell line | Drug | | Schedule | C.I. | Effect of combination |
| A2780 | Compound 1 µM | cisplatin µM | | | |
| | 0.5 | 10,000 | Simultaneous | 0.69 | Synergism |
| | 0.25 | 10,000 | | 0.73 | Synergism |
| | 0.5 | 10,000 | Sequential | 0.63 | Synergism |
| | 0.25 | 10,000 | | 0.74 | Synergism |
| HCT-116 | 0.5 | 10,000 | Sequential | 0.73 | Synergism |

### Example 3: In vitro cytotoxic activity of the combination with oxaliplatin

The results shown in Table 2 indicate that on human colon carcinoma HCT-116 cell line the administration of Compound 1 in combination with oxaliplatin resulted in a synergistic antitumor effect.

| **Table 2.** *In vitro* evaluation of the cytotoxic effect of Compound 1 in combination with oxaliplatin | | | | | |
|---|---|---|---|---|---|
| Cell line | Drug | | Schedule | C.I. | Effect of combination |
| HCT-116 | Compound 1 µM | oxaliplatin µM | | | |
| | 0.5 | 20,000 | Sequential | 0.58 | Synergism |
| | 0.25 | 20,000 | | 0.70 | Synergism |
| | 0.12 | 20,000 | | 0.79 | Synergism |

### Example 4: In vitro cytotoxic activity of the combination with 5-FU

The results shown in Table 3 indicate that on human colon carcinoma HCT-116 cell line the administration of Compound 1 in combination with 5-FU resulted in a synergistic antitumor effect.

| **Table 3.** *In vitro* evaluation of the cytotoxic effect of Compound 1 in combination with 5-FU | | | | | |
|---|---|---|---|---|---|
| Cell line | Drug | | Schedule | C.I. | Effect of combination |
| HCT-116 | Compound 1 µM | 5-FU nM | | | |
| | 0.5 | 12.5 | Sequential | 0.77 | Synergism |
| | 0.25 | 3.1 | | 0.76 | Synergism |

### Example 5: In vitro cytotoxic activity of the combination with SN38

The results shown in Table 4 indicate that on human ovarian carcinoma A2780 the administration of Compound 1 in combination with the topoisomerase I inhibitor SN38 resulted in a synergistic antitumor effect.

| **Table 4.** *In vitro* evaluation of the cytotoxic effect of Compound 1 in combination with SN38 | | | | | |
|---|---|---|---|---|---|
| Cell line | Drug | | Schedule | C.I. | Effect of combination |
| A2780 | Compound 1 µM | SN38 µM | | | |
| | 0.5 | 0.012 | Simultaneous | 0.78 | Synergism |
| | 0.25 | 0.012 | | 0.80 | Synergism |
| | 0.5 | 0.012 | Sequential | 0.58 | Synergism |
| | 0.25 | 0.012 | | 0.56 | Synergism |

### Example 6: In vitro cytotoxic activity of the combination with AraC

The results shown in Table 5 indicate that on human colon carcinoma HCT-116 cell line the administration of Compound 1 in combination with AraC resulted in a synergistic antitumor effect.

| **Table 5.** *In vitro* evaluation of the cytotoxic effect of Compound 1 in combination with AraC | | | | | |
|---|---|---|---|---|---|
| Cell line | Drug | | Schedule | C.I. | Effect of combination |
| HCT-116 | Compound 1 µM | AraC nM | | | |
| | 0.5 | 0.25 | Sequential | 0.46 | Synergism |
| | 0.25 | 0.25 | | 0.40 | Synergism |

### Example 7: In vivo antitumor efficacy in combination with gemcitabine

Balb, Nu/Nu male mice, from Harlan (Italy), were maintained in cages with paper filter cover, food and bedding sterilized and water acidified. Fragments of BX-PC3 human pancreatic carcinoma were implanted subcutaneously in athymic mice. This tumor model was selected because it was previously demonstrated that it is sensitive to gemcitabine and also because gemcitabine is the standard treatment for pancreatic cancer. The treatment with gemcitabine started at day 9 after tumor implantation, when tumors were palpable. Compounds were prepared immediately before treatments.

Compound 1 of formula (A) was administrated by intraperitoneal route in a volume of 10 ml/kg at the dose of 15 mg/kg twice a day (BID) for 9 consecutive days (days 9, 10, 11, 12, 13, 14, 15, 16 and 17). Gemcitabine was administered intravenously in a volume of 10 ml/kg at the dose of 80 mg/kg for 3 times every 4 days (at days 9, 13, 17). When combined, gemcitabine was administered at days 9, 13, 17 and compound 1 was administered for the following 3 days after gemcitabine administration. Tumor growth and body weight were measured every 3 days. Tumor growth was assessed by caliper. The two diameters were recorded and the tumor weight was calculated according the following formula: length (mm) x width²/2. The effect of the antitumor treatment was evaluated as percentage of tumor growth inhibition (TGI %). Toxicity was evaluated on the basis of body weight reduction. The results are reported in Table 6. Compound 1 combined with the antimetabolite agent gemcitabine produced an additive/synergic effect.

| **Table 6**. *In vivo* efficacy of the combination with gemcitabine | | |
|---|---|---|
| Treatment | TGI % | Toxicity |
| compound 1 | 53 | 0/8 |
| 15 mg/kg* | | |
| gemcitabine 80 mg/kg** | 61 | 0/8 |
| Gemcitabine 80 mg/kg + compound 1 | 70 | 0/8 |
| 15 mg/kg*** | | |

| | | |
|---|---|---|
| *Treatments made intraperitoneally twice at days 9-17 **Treatments made intravenously at days 9, 13, 17 *** gemcitabine treatments on day 9, 13, 17; compound 1 treatments on day 10, 11,12, 14, 15, 16, 18, 19, 20 | | |

### Example 8: In vivo antitumor efficacy in combination with cisplatin

Balb, Nu/Nu male mice, from Harlan (Italy), were maintained in cages with paper filter cover, food and bedding sterilized and water acidified. A2780 human ovarian carcinoma cells were obtained from American Type Culture Collection (Rockville, MD) and were maintained in vitro as continuous cultures in RPMI medium supplemented with 10% FBS, at 37 °C, 5% CO₂. For in vivo experiments 8x10⁶ A2780 cells were implanted subcutaneously in athymic mice. This tumor model was selected because it was sensitive to platinum derivatives and also on the basis of use of this drugs in ovarian cancer.

The treatment started when tumors were palpable (day 13). Both compounds were prepared immediately before treatment.

Compound of 1 of formula (A) was administered by intraperitoneal route in a volume of 10 ml/kg at the dose of 15 mg/kg twice a day (BID) from day 13 to day 17. Cisplatin was administered by intravenous route in a volume of 10 ml/kg at the dose of 8 mg/kg at day 5. When combined, compound 1 was administered from day 1 to day 5, cisplatin at day 17. Tumor growth and body weight were measured every 3 days. Tumor growth was assessed by caliper. The two diameters were recorded and the tumor weight was calculated according the following formula: length (mm) x width²/2. Animals were sacrificed on day 37 after tumor implant, day 24 from the start of treatments and tumors were weighted. The effect of the antitumor treatment was evaluated as percentage of tumor growth inhibition of the treated group respect to control. Toxicity was evaluated on the basis of body weigh reduction. The results are reported in Table 7.

| **Table 7**. *In vivo* efficacy of the combination with cisplatin | | |
|---|---|---|
| Treatment | TGI% | Toxicity |
| Compound 1 | 46 | 0/7 |
| 15 mg/kg* | | |
| Cisplatin 8 mg/kg | 21 | 0/7 |
| Cisplatin 8 mg/kg + Compound 1 | 77 | 0/7 |
| 15 mg/kg*** | | |

| | | |
|---|---|---|
| *Treatments made ip twice at days 13,14,15,16, 17. **Treatments made by intravenous route at days 17 *** Days 17: cisplatin treatments, days 13-17: Compound 1 treatments | | |

Compound 1 of formula (A) combined with the alkylating agent cisplatin produced a clear synergistic effect. No toxicity was observed in any of the treatment groups.

### Example 9: In vivo antitumor efficacy of the combination with irinotecan

Balb, Nu/Nu male mice, from Harlan (Italy), were maintained in cages with paper filter cover, food and bedding sterilized and water acidified. A2780 human ovarian carcinoma cells were obtained from American Type Culture Collection (Rockville, MD) and were maintained in vitro as continuous cultures in RPMI medium supplemented with 10% FBS, at 37 °C, 5% CO₂. For in vivo experiments 8x10⁶ A2780 were implanted subcutaneously in athymic mice. This tumor model was selected because it was sensitive to platinum derivatives and also on the basis of use of this drugs in ovarian cancer.

The treatment started when tumors were palpable (day 13). Both compounds were prepared immediately before treatment.

Compound 1 of formula (A) was administered by intraperitoneal route in a volume of 10 ml/kg at the dose of 30 mg/kg twice a day (BID) from day 13 to day 17. Irinotecan was administered by intravenous route in a volume of 10 ml/kg at the dose of 60 mg/kg at day 1. When combined, compound 1 was administered from day 1 to day 5, irinotecan at day 1. Tumor growth and body weight were measured every 3 days. Tumor growth was assessed by caliper. The two diameters were recorded and the tumor weight was calculated according the following formula: length (mm) x width²/2. Animals were sacrificed on day 37 after tumor implant, day 24 from the start of treatments and tumors were weighted. The effect of the antitumor treatment was evaluated as percentage of tumor growth inhibition of the treated group respect to control. Toxicity was evaluated on the basis of body weigh reduction. The results are reported in Table 8.

| **Table 8**. *In vivo* efficacy of the combination with irinotecan | | |
|---|---|---|
| Treatment | TGI% | Toxicity |
| Compound 1 30 mg/kg* | 72.3 | 0/7 |
| irinotecan 60 mg/kg** | 62 | 0/7 |
| irinotecan 50 mg/kg + Compound 1 30 mg/kg*** | 89.9 | 0/7 |

| | | |
|---|---|---|
| *Treatments made ip twice at days 13,14,15,16, 17. **Treatments made by intravenous route at days 13 *** Days 13: irinotecan treatments, days 13-17: Compound 1 treatments | | |

Compound 1 of formula (A) combined with the topoisomerase I inhibitor irinotecan produced a clear synergistic effect. No toxicity was observed in any of the treatment groups.

### Example 10: In vivo antitumor efficacy of the combination with docetaxel

Balb, Nu\Nu male mice, from Harlan (Italy), were maintained in cages with paper filter cover, food and bedding sterilized and water acidified. 2.5 x10⁶ DU145 prostate carcinoma cells (from the American Type Culture Collection) were injected subcutaneously in athymic mice. This tumor model was selected because it was previously demonstrated that docetaxel inhibits growth of the model in vivo (see for reference: Cancer Res. 2004 Oct 15, (64): 7426-31) and also on the basis of use of this drug in prostate cancer (see, for references, Approval summary: docetaxel in combination with prednisone for the treatment of androgen-independent hormone-refractory prostate cancer, Clin. Cancer Res. 2004 Dec 15; 10(24): 8147-51).

The treatment started 10 days later tumor cells injection when tumors were palpable. Docetaxel was prepared immediately before treatment, compound 1 of formula (A) was prepared daily, on the basis of known stability of the compound.

Compound 1 was administered by intraperitoneal route in a volume of 10 ml/kg at the doses of 30 mg\kg twice a day (BID) for 9 days (days 10 to 18). Docetaxel was administered by intravenous route in a volume of 10 ml/kg at the dose of 7.5 mg/kg on days 10, 14, 18 from the days of cell injection. When combined, compound 1 was administered in the interval between the docetaxel treatments at days 11, 12, 13, 15, 16, 17, 19, 20 and 21. Tumor growth and body weight were measured every 3 days. Tumor growth was assessed by caliper. The two diameters were recorded and the tumor weight was calculated according the following formula:
length (mm) x width²/2. The effect of the antitumor treatment was evaluated as the delay in the onset of an exponential growth of the tumor (see for references, Anticancer drugs 7:437-60, 1996). This delay (T-C value) was defined as the difference of time (in days) required for the treatment group (T) and the control group (C) tumors to reach a predetermined size (1g). Toxicity was evaluated on the basis of body weigh reduction. The results were reported in table below. Compound of formula (A) combined with docetaxel produced a clear synergistic effect: The T-C observed when compound 1 was combined with docetaxel was superior (11.5 days) to the expected by the simple addition of T-C (9) obtained by the single treatments. No toxicity was observed in any of the treatment groups. The results are reported in Table 9.

| **Table 9**. *In vivo* efficacy of the combination with docetaxel | | |
|---|---|---|
| Treatment | T-C (days) | Toxicity |
| Compound 1 30 mg/kg* | 5.3 | 0/7 |
| docetaxel 7.5 mg/kg** | 3.7 | 0/7 |
| docetaxel 7.5 mg/kg + Compound 1 30 mg/kg*** | 11.5 | 0/7 |

| | | |
|---|---|---|
| *Treatments made intraperitoneally twice at days 10-18. **Treatments made by intravenous route at days 10, 14, 18 *** Days 10, 14, 18: docetaxel treatments, days 11, 12, 13, 15, 16, 17, 19, 20 and 21: Compound 1 treatments | | |

## Claims

1. A therapeutic combination consisting of (a) Compound 1 of formula (A): 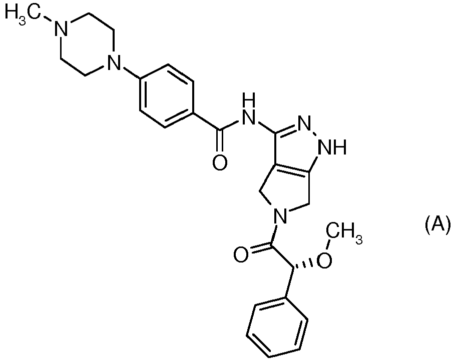 and (b) one or more antineoplastic agents selected from the group consisting of cisplatin, oxaliplatin, 5-fluorouracil, gemcitabine, cytosine arabinoside (Ara-C), SN-38, irinotecan and docetaxel, wherein the active ingredients of the combination are present in each case in free form or in the form of a pharmaceutically acceptable salt or any hydrate thereof.

2. A therapeutic combination consisting of (a) a compound 1 of formula (A): 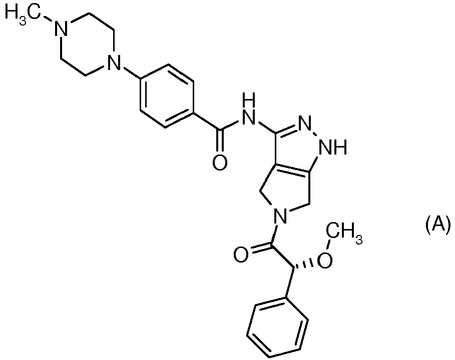 and (b) one or more antineoplastic agents selected from the group consisting of cisplatin, oxaliplatin, 5-fluorouracil, gemcitabine, cytosine arabinoside (Ara-C), SN-38, irinotecan and docetaxel for use in a method of treating or delaying the progression of a proliferative disorder, wherein the said method comprises a simultaneous, sequential or separate administration to a patient in need thereof of a therapeutically effective amount of the said combination.

3. A pharmaceutical composition comprising a combination according to claim 1 admixed with a pharmaceutically acceptable carrier, diluent or excipient.

## Patentansprüche

1. Therapeutische Kombination, bestehend aus (a) Verbindung 1 der Formel (A): 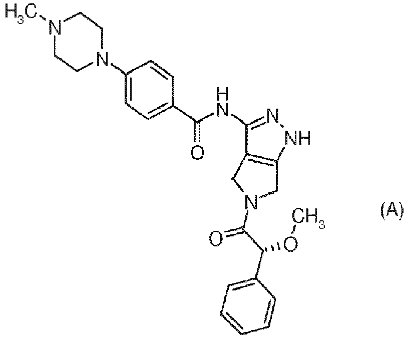 und (b) einem oder mehreren antineoplastischen Mitteln ausgewählt aus der Gruppe, bestehend aus Cisplatin, Oxaliplatin, 5-Fluoruracil, Gemcitabin, Cytosinarabinosid (Ara-C), SN-38, Irinotecan und Docetaxel, wobei die aktiven Bestandteile der Kombination jeweils in freier Form oder in Form eines pharmazeutisch verträglichen Salzes oder eines beliebigen Hydrats davon vorliegen.

2. Therapeutische Kombination, bestehend aus (a) einer Verbindung 1 der Formel (A): 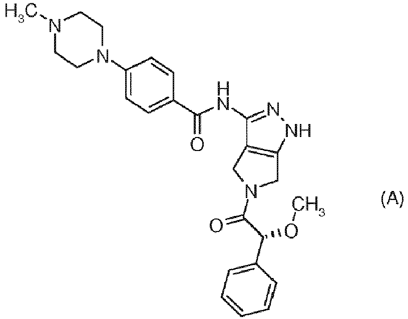 und (b) einem oder mehreren antineoplastischen Mitteln ausgewählt aus der Gruppe, bestehend aus Cisplatin, Oxaliplatin, 5-Fluoruracil, Gemcitabin, Cytosinarabinosid (Ara-C), SN-38, Irinotecan und Docetaxel zur Verwendung in einem Verfahren zur Behandlung oder Verzögerung der Progression einer proliferativen Störung, wobei das Verfahren eine gleichzeitige, aufeinanderfolgende oder separate Verabreichung einer therapeutisch wirksamen Menge der Kombination an einen Patienten umfasst, der dieser bedarf.

3. Pharmazeutische Zusammensetzung, die eine Kombination nach Anspruch 1 umfasst, gemischt mit einem pharmazeutisch verträglichen Träger, Verdünnungsmittel oder Exzipienten.

## Revendications

1. Combinaison thérapeutique constituée (a) du composé 1 de formule (A) : 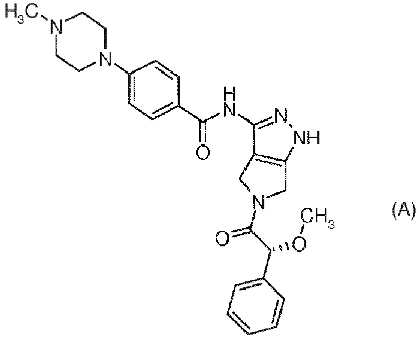 et (b) d'un ou plusieurs agents antinéoplasiques sélectionnés dans le groupe constitué du cisplatine, de l'oxaliplatine, du 5-fluorouracil, de la gemcitabine, du cytosine arabinoside (Ara-C), de SN-38, de l'irinotécane et du docétaxel, dans lequel les ingrédients actifs de la combinaison sont présents dans chaque cas sous la forme libre ou sous la forme d'un sel pharmaceutiquement acceptable ou de tout hydrate de ceux-ci.

2. Combinaison thérapeutique constituée (a) du composé 1 de formule (A) : 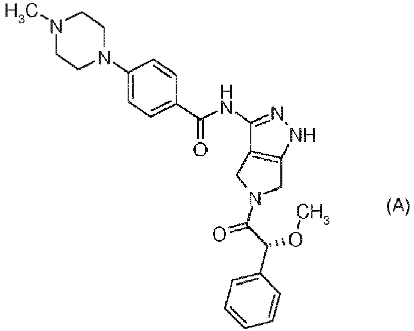 et (b) d'un ou plusieurs agents antinéoplasiques sélectionnés dans le groupe constitué du cisplatine, de l'oxaliplatine, du 5-fluorouracil, de la gemcitabine, du cytosine arabinoside (Ara-C), de SN-38, de l'irinotécane et du docétaxel pour son utilisation dans un procédé de traitement ou de ralentissement de la progression d'un trouble de la prolifération, dans lequel ledit procédé comprend une administration simultanée, séquentielle ou séparée à un patient le nécessitant d'une quantité thérapeutiquement efficace de ladite combinaison.

3. Composition pharmaceutique comprenant une combinaison selon la revendication 1 mélangée avec un vecteur, un diluant ou un excipient pharmaceutiquement acceptables.
